# FASCICULE DE BREVET EUROPEEN

(11) **EP 3 921 634 B1**
(45) Date de publication et mention de la délivrance du brevet: **10.12.2025**
(21) Numéro de dépôt: 20709273.5
(22) Date de dépôt: 04.02.2020
(51) Int. Cl.: G01N 33/00, G01N 33/24

(54) **DISPOSITIF DE MESURE DE LA CONCENTRATION D'UN GAZ DANS LE SOL D'UNE RÉGION, EN PARTICULIER DIHYDROGÈNE**
VORRICHTUNG ZUR MESSUNG DER KONZENTRATION EINES GASES IM BODEN EINES BEREICHS, INSBESONDERE VON DIWASSERSTOFF
DEVICE FOR MEASURING THE CONCENTRATION OF A GAS IN THE SOIL OF A REGION, IN PARTICULAR DIHYDROGEN

(30) Priorité: 05.02.2019 FR 1901121
(43) Date de publication de la demande: 15.12.2021
(73) Titulaire: ENGIE, 92400 Courbevoie (FR)
(72) Inventeur: GORINTIN, Louis, 92120 MONTROUGE (FR); WERLY, Julien, 95120 ERMONT (FR); D'AGOSTINO, Angélique, 94490 ORMESSON SUR MARNE (FR)
(74) Mandataire: Cabinet Beau de Loménie
(86) Numéro de dépôt international: PCT/FR2020/050183
(87) Numéro de publication internationale: WO 2020/161431

(56) Documents cités:
- EP-A1- 2 575 324
- WO-A1-2012/032306
- HIRATA TADAAKI ET AL: "Proposal of a power saving network for rice fields using LoRa", 2017 IEEE 6TH GLOBAL CONFERENCE ON CONSUMER ELECTRONICS (GCCE), IEEE, 24 October 2017 (2017-10-24), pages 1 - 4, XP033286903, [retrieved on 20171219], DOI: 10.1109/GCCE.2017.8229298
- SCHULZ GUNTHER: "Sync Node RTC to network server[Question] . Issue #300", 21 August 2017 (2017-08-21), pages 1 - 3, XP093052059, Retrieved from the Internet <URL:https://github.com/Lora-net/LoRaMac-node/issues/300> [retrieved on 20230606]
- DI MARTINO ROBERTO M R ET AL: "Continuous monitoring of hydrogen and carbon dioxide at Mt Etna", CHEMICAL GEOLOGY, ELSEVIER SCIENCE PUBLISHER B.V., AMSTERDAM, NL, vol. 357, 20 August 2013 (2013-08-20), pages 41 - 51, XP028763142, ISSN: 0009-2541, DOI: 10.1016/J.CHEMGEO.2013.08.023
- GUOYING WANG ET AL: "SCFSen: A Sensor Node for Regional Soil Carbon Flux Monitoring", SENSORS, vol. 18, no. 11, 16 November 2018 (2018-11-16), pages 1 - 19, XP055642370, DOI: 10.3390/s18113986
- GUOYING WANG ET AL: "Time Domain Similarity of Lightweight Parameters Based Soil Respiration Sensor Network Deployment", INTERNATIONAL JOURNAL OF DISTRIBUTED SENSOR NETWORKS, vol. 11, no. 6, 530272, 1 January 2015 (2015-01-01), pages 1 - 13, XP055642374, ISSN: 1550-1477, DOI: 10.1155/2015/530272

## Description

### Domaine Technique

L'invention se rapporte au domaine général des dispositifs électroniques équipés de capteurs, par exemple des dispositifs de mesure. En particulier, l'invention concerne les dispositifs électroniques équipés de capteurs configurés pour détecter des fluides (liquides/gaz) dans le sol d'une région.

L'invention concerne en particulier la détection de dihydrogène dans le sol.

### Technique antérieure

Le sol de certaines régions contient naturellement différentes sortes de gaz, et en particulier du dihydrogène. La production de ce dihydrogène pourrait être liée à une réaction ferrique de l'eau à haute température (Oxydation de roche ferreuse à haute température et haute pression, ce qui peut émettre du dihydrogène), selon une hypothèse.

En tout état de cause, il est particulièrement intéressant de déterminer où sont les poches souterraines de dihydrogène dans le sol d'une région et il a donc été proposé d'effectuer des mesures ponctuelles qui donnent une valeur de concentration de dihydrogène dans le sol.

L'appareil ayant pour référence GA5000 et commercialisé par la société britannique Geotech convient pour ce type de mesures. Cet appareil est équipé d'un capteur qui détecte la concentration de dihydrogène dans les gaz. Pour utiliser cet appareil, un opérateur fore le sol pour y installer une canne de prélèvement à laquelle on connecte cet appareil. L'opérateur peut lire sur l'appareil la concentration de dihydrogène.

Cette solution n'est pas satisfaisante, car elle requiert la présence d'un opérateur pour lire les données sur le dispositif. Il n'est pas non plus possible de mesurer une évolution sur de longues périodes (de quelques jours à plusieurs mois) de la concentration de dihydrogène.

Il est également particulièrement délicat, avec cet appareil connu, d'obtenir une cartographie bidimensionnelle de la concentration en dihydrogène dans le sol d'une région : cela requiert la présence d'un opérateur à chaque point de mesure.

Si l'utilisation de l'appareil GA5000 permet d'avoir une information ponctuelle sur la quantité de dihydrogène présent dans le sol, il a également été proposé des méthodes qualitatives. En effet, il a été observé que certains gaz empêchent la croissance de végétaux : des zones circulaires sans végétaux peuvent ainsi être observées (typiquement avec un diamètre de plusieurs centaines de mètres).

Par exemple, cela peut être implémenté ainsi :
- à courte distance : à l'œil nu, un opérateur peut observer le sol et les végétaux d'une zone ;
- à moyenne distance, un drone équipé d'une caméra peut faciliter la détection de zones circulaires sans végétaux ;
- à longue distance, des structures circulaires de 500 à 1000 mètres de diamètre peuvent être détectées par des images satellites, en se focalisant sur l'émission de certaines longueurs d'onde.

Comme on le conçoit, la détection de ces zones ne fournit pas d'informations sur la quantité de gaz présente dans le sol.

Des méthodes similaires sont basées sur l'utilisation au sol de caméras spectrales pour détecter les gaz dans l'air (mais cette méthode ne fonctionne pas pour les gaz dans le sol).

On connait également des méthodes dans lesquelles on va mesurer des vibrations sismiques par un réseau de capteurs communicants, mais cette solution ne permet pas de détecter la présence de gaz produit si cette production ne produit pas également de vibrations sismiques. La société américaine Wireless Seismic, Inc. produit de tels systèmes. De l'état de la technique antérieure, on connait également les documents suivants :
- « Continuous monitoring of hydrogen and carbon dioxide at Mt Etna » (Di Martino et al, CHEMICAL GEOLOGY, vol. 357, 20 août 2013, pages 41-51),
- « SCFSen : A Sensor Node for Regional Soil Carbon Flux Monitoring » (Wang et al, SENSORS, vol. 18, 16 novembre 2018, pages 1-19),
- « Time Domain Similarity of Lightweight Parameters Based Soil Respiration Sensor Network Deployment » (Wang et al, INTERNATIONAL JOURNAL OF DISTRIBUTED SENSOR NETWORKS, vol. 11, 1er janvier 2015, article numéro 530272, pages 1-13),
- WO 2012/032306, et
- EP 2 575 324.

Les solutions existantes ne permettent pas de mesurer facilement la concentration d'un fluide, typiquement du dihydrogène gazeux, dans le sol d'une région. Elles rendent difficile l'élaboration de cartographies bidimensionnelles sur lesquelles on peut lire la concentration d'un fluide particulier. Les mêmes problèmes se posent pour des mesures d'autres caractéristiques de fluides.

L'invention vise notamment à pallier ces inconvénients.

### Exposé de l'invention

A cet effet, l'invention propose un dispositif de mesure (pouvant aussi être appelé détecteur), comprenant au moins :
- un capteur configuré pour mesurer une caractéristique d'un gaz, la caractéristique étant la concentration du gaz dans le sol d'une région,
- un module de communication sans-fil configuré pour émettre la caractéristique mesurée du gaz.

Ainsi, l'invention permet d'automatiser la collecte d'informations provenant d'un capteur, car elle est équipée d'un module de communication sans-fil.

On peut noter que le dispositif peut comporter un microcontrôleur apte à stocker dans une mémoire (non-volatile) interne ou externe les caractéristiques mesurées. Ce contrôleur peut être utilisé pour déclencher la mesure et l'émission de la caractéristique mesurée.

Selon l'invention, le dispositif comprend une horloge, et un module de contrôle du capteur configuré pour que le capteur mesure la caractéristique mesurée pendant une plage de mesure prédéterminée (par exemple une heure prédéterminée).

Cette horloge peut être un composant bien connu de l'homme du métier sous l'acronyme anglo-saxon « RTC : real-time clock », et pourra être choisi de manière à minimiser la dérive de l'horloge.

Ce mode selon l'invention est avantageux si plusieurs dispositifs sont utilisés au sein d'une même région, on peut ainsi synchroniser les mesures pour qu'elles soient toutes mises en oeuvre dans la même plage de mesure (par exemple à partir d'une même heure prédéterminée), chaque dispositif utilisant sa propre horloge à cet effet.

Selon l'invention, le module de communication sans-fil est configuré pour émettre ladite caractéristique mesurée pendant une plage d'émission prédéterminée (par exemple à une heure prédéterminée).

Cette horloge peut être analogue à celle utilisée pour déterminer lesdites plages de mesure, voire être la même horloge. Elle peut donc être également un composant bien connu de l'homme du métier sous l'acronyme anglo-saxon « RTC : real-time clock », et qui pourra être choisi de manière à minimiser la dérive de l'horloge.

Eventuellement, l'horloge est interne audit microcontrôleur.

On peut noter que la valeur de la caractéristique mesurée peut par exemple être émise de manière régulière grâce à l'horloge : par exemple toutes les heures ou tous les jours.

En outre, l'utilisation de l'horloge permet de choisir une plage d'émission ou une heure d'envoi qui sera celle d'écoute d'un autre appareil, par exemple un concentrateur.

On peut noter que la plage de mesure précède la plage d'émission, et que chaque plage d'émission peut être associée à une plage de mesure.

Selon l'invention, le module de communication sans-fil est configuré pour communiquer avec un concentrateur.

Le concentrateur peut communiquer avec plusieurs dispositifs électroniques tels que celui décrit ci-avant. Il peut notamment être capable de communiquer à travers un réseau de communication tel que l'Internet, pour rendre accessibles les informations relevées par les dispositifs électroniques (les caractéristiques mesurées).

Selon l'invention, si plusieurs dispositifs de mesure équipés d'horloges sont utilisés, alors ils émettent leurs caractéristiques mesurées respectives à des plages d'émissions ou heures qui sont toutes différentes. Ainsi, il n'y a pas de collisions entre des messages envoyés par différents dispositifs de mesure. Cela permet au concentrateur de ne pas être de manière permanente dans un état d'écoute, et ainsi de consommer moins d'énergie électrique : cela favorise donc son autonomie énergétique.

L'utilisation de liaisons sans fil à faible consommation énergétique utilisant le protocole LoRaWAN dans sa version 1.1, ou l'un des protocoles utilisables sur un réseau « LPWAN : Low-Power Wide-Area Network » dédiés à l'Internet des Objets, permet aux dispositifs de consommer très peu d'énergie et donc d'être alimentés avec uniquement un module d'alimentation en énergie électrique de type batterie, pile, ou encore par des panneaux photovoltaïques.

Selon un mode de réalisation particulier, ladite horloge est configurée pour être synchronisée sur la base d'un message émis par le concentrateur et reçu par le module de communication sans-fil (du dispositif de mesure).

Par exemple, le message peut contenir l'heure actuelle du concentrateur.

Ce mode de réalisation particulier est particulièrement utile lorsque plusieurs dispositifs de mesure sont utilisés : ils sont ainsi tous synchronisés sur l'horloge du concentrateur qui leur émet à tous son heure actuelle dans un message.

Selon un autre exemple, lors de la première communication entre le dispositif de mesure et le concentrateur, le concentrateur peut émettre ledit message au dispositif. Cette émission peut avoir lieu lorsque le concentrateur identifie le dispositif.

Aussi, le message émis par le concentrateur peut être un message de correction de dérive de l'horloge.

Selon l'invention, le dispositif est configuré pour émettre, avec ladite caractéristique mesurée, un instant d'émission (typiquement l'heure dans la journée) de la caractéristique mesurée (par exemple déterminée au moyen de l'horloge).

Cette horloge peut être analogue à celle utilisée pour déterminer lesdites plages de mesure ou lesdites plages d'émission, voire être la même horloge.

Sur la base de l'instant d'émission de la caractéristique mesurée, le concentrateur peut déterminer une dérive de l'horloge du dispositif par rapport à sa propre horloge et déclencher l'envoi d'un message de correction de dérive au dispositif.

Ainsi, selon un mode de réalisation particulier, le dispositif est configuré pour corriger une dérive de son horloge sur la base d'un message émis par le concentrateur (typiquement, il contient une durée positive ou négative).

Selon un mode de réalisation particulier, le dispositif comporte :
- une canne tubulaire équipée d'un ou de plusieurs orifices traversant la paroi de la canne tubulaire, ledit un ou lesdits plusieurs orifices étant placés à proximité d'une extrémité de la canne tubulaire destinée à être insérée dans le sol, et une pompe pour aspirer le fluide présent dans le sol à travers le ou les orifices et ladite canne tubulaire, le fluide aspiré étant reçu par le capteur.

Ainsi, ce mode de réalisation particulier est particulièrement bien adapté à la détection de gaz produits dans le sol d'une région. Le capteur reçoit le fluide aspiré par la pompe pour mesurer la caractéristique.

Selon un mode de réalisation particulier, la canne a une longueur supérieure à 10 ou 70 centimètres, et inférieure 1, 3, ou 10 mètres.

Les inventeurs de la présente invention ont observé qu'avec une canne ayant une longueur comprise entre 70 centimètres et 1 mètre, on obtient de bons résultats. La longueur de la canne est mesurée entre l'extrémité destinée à être insérée et son extrémité qui peut être connectée à la pompe.

Selon un mode de réalisation particulier, la canne comprend entre 10 et 100 orifices traversant la paroi de la canne.

En particulier, l'homme du métier pourra choisir un nombre d'orifices capable de faire passer un débit maximal de 500 millilitres par minute (par exemple), et ce en minimisant la perte de charge.

Selon un mode de réalisation particulier, le ou les orifices ont un diamètre compris entre 1 et 3 millimètres, par exemple de 2 millimètres.

Selon un mode particulier de réalisation, les orifices sont espacés les uns par rapport aux autres d'au moins une distance comprise entre 1 et 3 millimètres, par exemple 2 millimètres.

Selon un mode particulier de réalisation, le ou les orifices sont agencés dans une portion de la canne s'étendant depuis ladite extrémité destinée à être insérée dans le sol sur toute la longueur de la canne ou sur une longueur comprise entre 3 millimètres et 20 centimètres.

Ces trois précédents modes de réalisation particuliers permettent de mesurer la caractéristique du fluide en un point bien précis du sol de la région.

Lorsque le ou les orifices sont agencés dans une portion de la canne s'étendant depuis ladite extrémité destinée à être insérée dans le sol sur une longueur comprise entre 3 millimètres et 20 centimètres. On mesure la caractéristique en un point ou en une localisation encore plus précisément.

Selon un mode de réalisation particulier, le dispositif comprend un module de communication supplémentaire configuré pour fournir une interface de commande à un utilisateur.

Par exemple, ce module de communication supplémentaire peut comporter une interface filaire ou une interface sans-fil de type Bluetooth. Cela peut permettre à un opérateur d'identifier le dispositif (par exemple au moyen d'un identifiant) et de commander cet appareil. Cela est particulièrement utile lors de l'installation de l'appareil.

Selon un mode de réalisation particulier, le dispositif comprend des filtres étanches aux liquides et perméables aux gaz agencés de part et d'autre dudit capteur.

Par exemple, ces filtres étanches aux liquides et perméables aux gaz peuvent comporter du polytétrafluoroéthylène (plus connu sous l'acronyme PTFE). L'utilisation de ces filtres est particulièrement adaptée pour mesurer des caractéristiques de fluides sous forme de gaz, et ils protègent le capteur.

Selon l'invention, le capteur mesure une concentration de molécules d'intérêt dans le gaz.

Selon un mode de réalisation particulier, le capteur est configuré pour détecter une concentration de dihydrogène.

Selon un mode de réalisation particulier, le module de communication sans-fil est un module compatible et conforme aux protocoles utilisés dans les réseaux de communication LPWAN, par exemple avec le protocole LoRaWAN ou le protocole Sigfox.

Selon un mode particulier de réalisation, le dispositif comprend un module d'alimentation en énergie électrique (préférentiellement portable).

Ce mode de réalisation particulier favorise l'autonomie du dispositif. On pourra notamment utiliser une réserve électrochimique (par exemple une pile), ou un module à panneaux photovoltaïques associé à une batterie, comme module d'alimentation en énergie électrique. L'homme du métier saura dimensionner cette source d'alimentation en fonction de l'application, c'est-à-dire en fonction de la durée d'utilisation du dispositif (par exemple un an).

Selon un mode de réalisation particulier, le dispositif comprend en outre un capteur de température configuré pour mesurer la température du fluide (éventuellement aspiré) et / ou un capteur d'humidité configuré pour mesurer l'humidité du fluide (éventuellement aspiré).

Les inventeurs de la présente invention ont observé que la température et/ou l'humidité (ou hygrométrie) ont une influence sur la sensibilité du détecteur vis-à-vis des molécules présentes dans le fluide à caractériser. Une phase préalable de calibration peut alors être mise en oeuvre.

L'invention propose également un ensemble comprenant au moins deux dispositifs tels que définis ci-avant. En particulier, cet ensemble peut comprendre des dispositifs selon tous les modes de réalisation particuliers décrits ci-avant.

Par exemple, l'ensemble peut comporter plusieurs dizaines desdits dispositifs, voire une centaine desdits dispositifs.

Selon un mode de réalisation particulier, l'ensemble comprend en outre un concentrateur.

Ce concentrateur peut être analogue à celui décrit ci-avant.

L'invention propose également une installation comprenant, au sein d'une région, une pluralité de dispositifs tels que décrits ci-avant agencés en des emplacements respectifs de la région (typiquement des emplacements tous différents), éventuellement avec leurs cannes tubulaires respectives insérées (au moins partiellement) dans le sol de la région. Par exemple, les dispositifs peuvent être agencés en des emplacements prédéfinis de la région, avec leurs cannes insérées dans le sol.

Selon un mode particulier de réalisation, les dispositifs sont agencés pour former un maillage sensiblement régulier.

Typiquement, on pourra former un maillage avec un espacement régulier entre les dispositifs. On pourra aussi placer les dispositifs le long d'un contour (par exemple circulaire) tous espacés régulièrement les uns après les autres.

Selon un mode de réalisation particulier, les dispositifs sont équipés d'horloges, et d'un module de contrôle de leur capteur configuré pour que les capteurs de tous les dispositifs mesurent la caractéristique mesurée du fluide pendant une même plage de mesure prédéterminée (par exemple au même instant).

Selon un mode de réalisation particulier, les dispositifs sont équipés d'horloges, les modules de communication sans-fil respectifs étant configurés pour émettre ladite caractéristique mesurée à des plages d'émissions (ou instants) prédéterminées (éventuellement pour des mesures mises en œuvre dans une même plage de mesure - déterminée avec l'horloge de chaque dispositif).

Préférentiellement, les plages d'émission sont toutes différentes. De manière alternative, les plages d'émission peuvent se chevaucher mais dans ce cas les bandes de fréquence utilisées pour deux plages d'émission se chevauchant ne se recouvrent pas.

Ce mode de réalisation particulier est particulièrement bien adapté pour l'utilisation de liaisons sans fil à faible consommation énergétique (mieux connues sous l'acronyme anglo-saxon « LPWAN : low-power wide-area network »), ou encore l'utilisation du protocole LoRaWAN.

Selon un mode de réalisation particulier, l'installation peut comprendre en outre un concentrateur.

Selon un mode de réalisation particulier, le concentrateur est configuré pour transmettre lesdites caractéristiques mesurées à un serveur distant.

Par exemple, cette transmission à un serveur distant peut être mise en oeuvre au moyen d'une liaison satellitaire.

Le serveur distant pourra notamment permettre à des utilisateurs d'accéder, par exemple au moyen du réseau internet, aux caractéristiques mesurées. En outre, le serveur distant pourra élaborer des cartographies bidimensionnelles des caractéristiques mesurées dans la région.

En fait, le concentrateur peut être configuré pour communiquer avec lesdits dispositifs, stocker les caractéristiques mesurées reçues, et les transférer à un serveur distant via l'Internet, éventuellement au moyen d'une liaison satellitaire.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
[Fig. 1] La figure 1 est une photographie d'une zone dans laquelle du dihydrogène produit dans le sol empêche la végétation de pousser.
[Fig. 2] La figure 2 est une représentation schématique d'un dispositif selon un exemple.
[Fig. 3] La figure 3 est une représentation schématique d'un dispositif muni d'une canne selon un exemple.
[Fig. 4] La figure 4 est une représentation schématique d'un dispositif muni d'une canne selon un autre exemple.
[Fig. 5] La figure 5 représente la communication entre des dispositifs et un concentrateur.
[Fig. 6] La figure 6 est un graphique qui représente le fonctionnement du capteur.

### Description des modes de réalisation

On va maintenant décrire des exemples de dispositifs communicants capables de mesurer la concentration du dihydrogène dans le sol et de communiquer cette concentration à un concentrateur.

L'invention n'est néanmoins nullement limitée à la détection de dihydrogène et s'applique à d'autres fluides d'intérêt à rechercher.

En fait, elle s'applique à toute caractéristique d'un fluide mesurable au moyen d'un capteur.

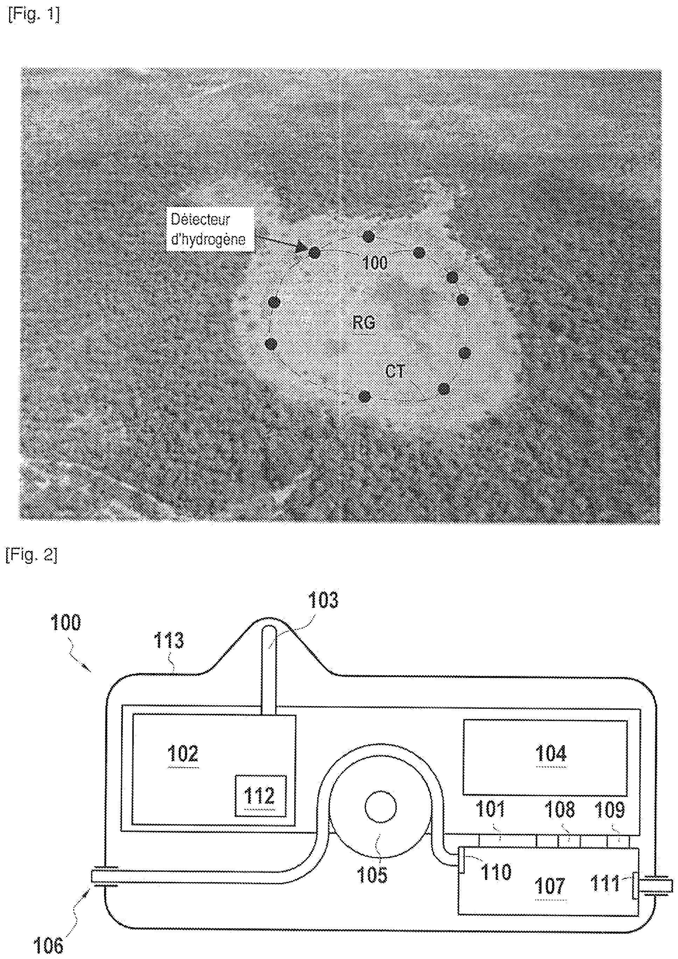

La figure 1 est une photographie prise depuis le ciel d'une région RG ayant une forme sensiblement circulaire et dans laquelle on observe une absence de végétation. Il a été observé qu'une telle zone circulaire peut résulter de la production souterraine de dihydrogène.

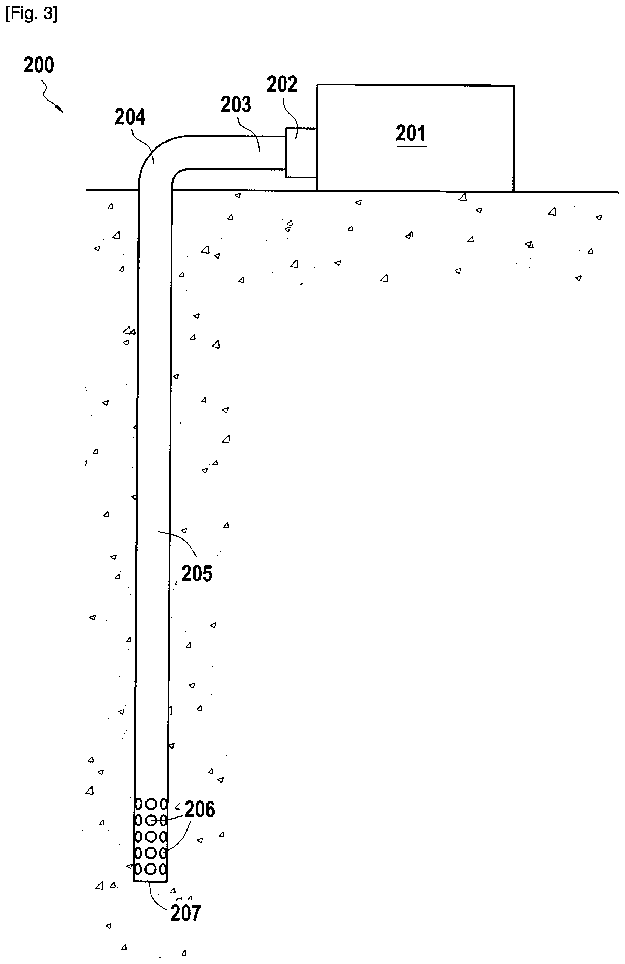

L'invention s'applique à ce type de région. Dans la région RG, on peut placer une pluralité de dispositifs 100 équipés de capteurs et de cannes (car la production de dihydrogène est ici souterraine). Ces dispositifs seront décrits plus en détail en référence aux figures 2 et 3.

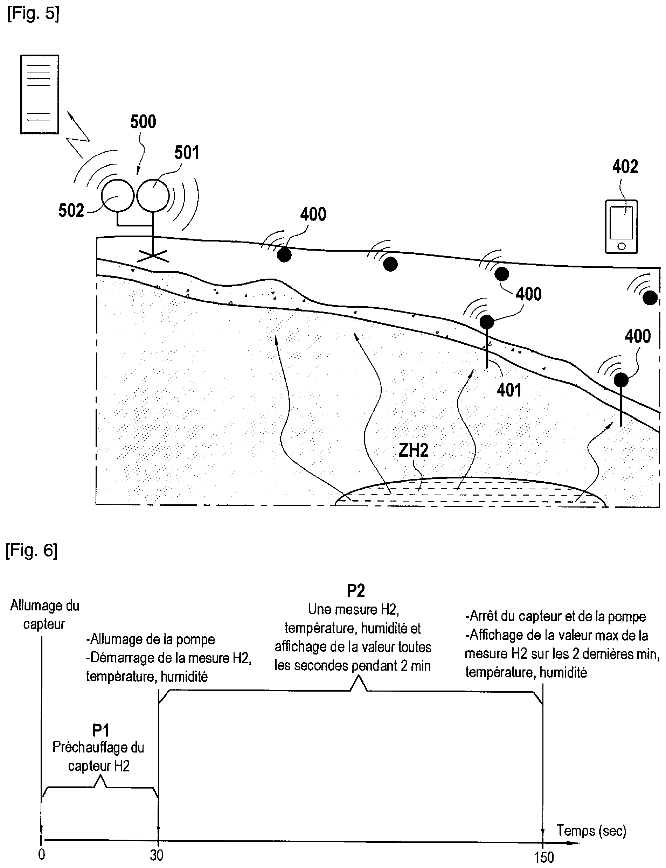

Pour les régions circulaires du type de la région RG, il a été observé par les inventeurs de la présente invention que la concentration de dihydrogène est plus élevée en périphérie qu'au centre de la région circulaire. De ce fait, et comme représenté sur la figure, on peut placer les dispositifs le long d'un contour CT de forme sensiblement circulaire avec les dispositifs tous espacés (sensiblement régulièrement) les uns après les autres. On pourra néanmoins placer un dispositif concentrateur, dont le fonctionnement sera décrit plus en détail en référence à la figure 5 décrite ci-après, par exemple au centre de la région circulaire RG.

En utilisant plusieurs dispositifs agencés le long du contour CT, on peut obtenir une cartographie bidimensionnelle, associée à un instant ou à une heure (on peut également parler de cartographie temporelle) de la concentration en dihydrogène, dans la mesure où les capteurs fonctionnent de manière simultanée, comme cela sera décrit ci-après.

Sur la figure 2, on a représenté de manière schématique un dispositif 100, pouvant être utilisé dans une région RG telle que celle de la figure 1. Le dispositif 100 comporte un capteur 101 de dihydrogène, par exemple du type électrochimique. Ce capteur peut mesurer une concentration de dihydrogène gazeux se trouvant dans un flux de gaz qui passe devant lui. La valeur de la concentration est mesurée sur commande d'une carte électronique 102 qui comporte un microcontrôleur, et également une mémoire non volatile (interne ou externe au microcontrôleur). Les valeurs de concentration mesurées par le capteur peuvent être enregistrées dans cette mémoire non volatile.

Le dispositif est également équipé d'une antenne 103 formant un module de communication sans-fil, ici selon le protocole LoRaWan dans sa version 1.1.L'antenne est également commandée par le microcontrôleur.

Pour alimenter en énergie électrique les différents composants du dispositif 100, on utilise une pile 104, préférablement dimensionnée pour que le dispositif puisse fonctionner au moins pendant un an.

Pour amener le fluide au capteur 101, on utilise une pompe 105, ici une pompe à membrane ou péristaltique, typiquement une pompe avec un débit réglable (par exemple de 40 à 500 millilitres par minute). Le fluide provient de l'entrée 106 par une connexion fluidique, et cette entrée peut être laissée libre si du dihydrogène présent en surface doit être détecté ou connectée à une canne, comme cela sera représenté sur la figure 3. La pompe 105 amène le fluide pompé dans une chambre 107 dans laquelle le capteur 101 a été placé.

D'autres capteurs sont placés dans la chambre 107, ici un capteur de température 108 et un capteur d'humidité 109. Il a été observé par les inventeurs que la température et l'humidité peuvent provoquer des dérives dans la concentration du dihydrogène mesuré, dès lors, en mesurant la température et l'humidité on peut corriger ces dérives (par exemple au sein du microcontrôleur).

De manière à protéger le capteur 101 contre les fluides qui peuvent l'empêcher de fonctionner (typiquement les liquides) et contre toute pollution, on a placé un filtre 110 à l'entrée de la chambre 107 et un filtre 111 à la sortie de la chambre 107. Les filtres 110 et 111 peuvent comprendre du PTFE, ce matériau étant étanche aux liquides et perméable aux gaz.

Le capteur 101 n'est pas destiné à fonctionner de manière continue, car cela diminuerait son autonomie et déchargerait la pile 104 trop rapidement. A cet effet, le dispositif est équipé d'une horloge 112, ici agencée dans la carte électronique 102 (éventuellement, l'horloge est dans le microcontrôleur). L'horloge 112 est un composant de type RTC. Selon l'invention, cette horloge déclenche la mesure du dihydrogène dans une plage de mesure prédéterminée, par exemple au début de chaque heure. Elle déclenche aussi la transmission au concentrateur de la concentration de dihydrogène dans une plage d'émission prédéterminée. Selon l'invention, l'heure à laquelle l'émission est faite est envoyée avec la concentration, car cela permet au concentrateur de corriger une dérive de l'horloge.

Enfin, le dispositif 100 est équipé d'un boîtier 113, par exemple en plastique, qui va protéger les composants 101 à 112.

Sur la figure 3, on a représenté un dispositif 200 comprenant un boîtier 201 qui est analogue au boîtier 113 décrit en référence à la figure 2. Ici, le boîtier 201 comprend un raccord 202 par clipsage ou vissage (par exemple) pour qu'un tube 203 s'y raccorde. Le tube 203 peut avoir une longueur de l'ordre de 10 centimètres.

Ici, le boîtier 201 est placé sur le sol d'une région. Comme on souhaite mesurer une concentration de dihydrogène provenant du sol, le tube 203 est connecté par un coude 204 à une canne 205 qui est insérée dans le sol à l'emplacement où l'on souhaite mesurer la concentration de dihydrogène.

La canne 205 a, dans l'exemple illustré, une longueur de 80 centimètres. La canne est tubulaire et sa paroi comporte des orifices traversants 206. Ces orifices traversants sont agencés régulièrement (ou non) sous la forme d'un quadrillage avec un espacement entre les orifices d'au moins 2 millimètres et un diamètre de 2 millimètres. Les orifices 206 sont tous agencés dans une portion de la canne 205 s'étendant depuis son extrémité insérée dans le sol 207 sur 10 centimètres. Cela permet de s'assurer que la mesure est bien effectuée à l'emplacement prédéterminé du sol de la région.

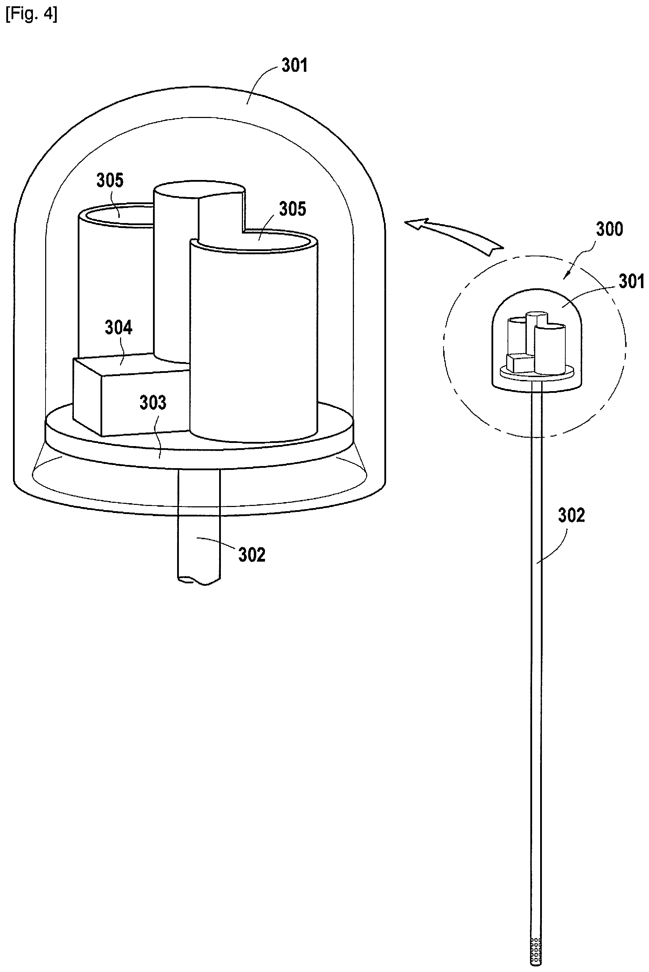

La figure 4 est une variante du dispositif de la figure 3. Le dispositif 300 de la figure 4 est comprend lui aussi un boîtier 301 destiné à être placé sur le sol d'une région, mais il est placé directement au-dessus de sa canne.

En fait, le boîtier 301 comprend un dôme comprenant un matériau de protection, typiquement en plastique. Une portion inférieure du boîtier, située sous le dôme, peut être en aluminium pour obtenir un bon blindage (à titre d'exemple, elle peut être cylindrique).

En outre, pour sa fixation à la canne 302, une embase 303, par exemple en aluminium, est agencée à la base du boîtier. Un joint torique peut alors être utilisé pour s'assurer que le boîtier est étanche, et on peut lier le boîtier à l'embase au moyen d'un filetage ou d'une fixation au quart de tour.

La pompe 304 est placée directement sur l'embase 303, et on a également prévu deux emplacements 305 aptes à recevoir des piles.

Sur la figure 5, on a représenté une pluralité de dispositifs 400, par exemple analogues aux dispositifs 200 ou 300 décrits ci avant. Les dispositifs 400 sont munis de cannes 401 insérées dans le sol d'une région, au-dessus d'une zone ZH2 contenant du dihydrogène qui peut diffuser vers la surface.

On peut noter que les dispositifs 400 sont tous équipés d'un module de communication Bluetooth. Cela peut permettre à un opérateur d'utiliser un appareil de type smartphone (c'est-à-dire un ordiphone) ou tablette 402 et d'identifier le dispositif (par exemple au moyen d'un identifiant) et de commander cet appareil. Cela est particulièrement utile lors de l'installation de l'appareil, mais également pour mettre en œuvre des relevés ponctuels. Par exemple, lorsqu'un utilisateur de ce type d'appareil communique avec un dispositif 400, éventuellement après que ce dispositif 400 a été sélectionné si plusieurs dispositifs 400 sont détectés, on peut lire sur l'écran de l'appareil 402 des informations choisies parmi : l'identifiant du dispositif, la localisation GPS du dispositif (s'il est équipé d'un module de localisation GPS), la valeur de la tension et l'état de charge de la pile, un ou plusieurs boutons pour sélectionner un mode de fonctionnement du dispositif (mesure ponctuelle ou régulière), le débit de la pompe (on peut éventuellement le régler), la fréquence de la mesure si elle est régulière, un bouton pour démarrer une mesure, les résultats des mesures, la concentration maximale de dihydrogène mesurée, les résultats des mesures de dihydrogène, de température, et d'humidité, avec l'heure des mesures, etc.

Ici, les dispositifs 400 communiquent avec un concentrateur 500. Cette communication est mise en oeuvre au moyen du protocole LoRaWAN et le concentrateur 500 comprend un module 501 de communication selon ce protocole. Il comporte également une horloge (non représentée ici).

Lors de la première communication entre chaque dispositif 400 et le concentrateur 500, le concentrateur 500 va envoyer, lors de sa première communication avec le détecteur, un message comprenant son heure actuelle (celle d'envoi du message) pour que les dispositifs 400 soient tous synchronisés.

Cette synchronisation permet aux dispositifs de tous mesurer la concentration de dihydrogène à un même instant, par exemple au début de chaque heure : c'est cela qui permet ensuite d'élaborer des cartographies bidimensionnelles et temporelles de la concentration en dihydrogène dans le sol de la région.

Selon l'invention, les émissions des concentrations, par les dispositifs 400, sont mises en œuvre à des instants prédéterminés et tous différents (en particulier si une seule bande de fréquence est utilisée). Dès lors, le concentrateur va écouter successivement à des plages horaires prédéterminées les messages provenant des dispositifs. Cela permet d'économiser de l'énergie et favorise l'autonomie du concentrateur.

A titre indicatif, si trois dispositifs sont utilisés, on peut mettre en œuvre la mesure au début de chaque heure, puis chacun à leur tour les dispositifs vont émettre la concentration avec leur instant d'émission à des heures prédéterminées, par exemple 15 minutes après le début de l'heure, 30 minutes après le début de l'heure, et 45 minutes après le début de l'heure.

Le concentrateur reçoit donc les concentrations avec les instants d'émission, qu'il peut comparer avec sa propre horloge ; alternativement, le concentrateur compare les heures ou instants de réception des concentrations avec une heure attendue pour ce message.

Dès lors, le concentrateur peut détecter une dérive dans l'horloge d'un des dispositifs 400, puis envoyer un message de correction de dérive avec notamment la valeur de la compensation à appliquer. Sur réception de ce message, les dispositifs 400 peuvent corriger la dérive de leur horloge.

Ainsi, on obtient une synchronisation améliorée des mesures et des envois pour garantir à la fois l'obtention de cartographies bidimensionnelles montrant la concentration de dihydrogène à un instant, et la faible consommation en énergie électrique des dispositifs et du concentrateur.

Aussi, le concentrateur 500 est muni d'un module 502 de communication à l'Internet. Par exemple, une liaison satellitaire ou une liaison par GSM/GPRS peut être utilisée à cet effet. Cela rend accessible à distance, à travers un serveur, les concentrations de dihydrogène mesurées dans la région.

Sur la figure 6, on a représenté les différentes phases d'une mesure de dihydrogène, mises en œuvre par l'un des dispositifs tel que décrit ci-avant.

Ces phases sont synchronisées comme expliqué ci-avant pour que plusieurs dispositifs les mettent en œuvre simultanément.

Dans une première phase P1, qui commence avec l'allumage du capteur, on met en œuvre un préchauffage du capteur. Cette première phase peut durer 30 secondes.

Ensuite, pendant une deuxième phase P2 ayant par exemple une durée de 120 secondes, on met en œuvre des mesures de dihydrogène, par exemple à la fréquence d'échantillonnage du capteur. Tous les résultats des mesures peuvent être envoyés au microcontrôleur du dispositif.

A la fin de la phase P2, on détermine, au moyen du microcontrôleur, la valeur maximale mesurée de concentration de dihydrogène. Cette valeur maximale devient la valeur à émettre au concentrateur. Avant cette émission, elle est mémorisée.

De manière alternative, on peut aussi déterminer la valeur moyenne des mesures pendant la phase P2, cette valeur moyenne devenant alors la valeur à émettre au concentrateur.

On peut également et de la même manière obtenir des maximums de température et d'humidité mesurés pendant la phase P2.

On notera que dans la présente demande, l'expression dispositif de mesure peut être remplacée par détecteur.

## Revendications

1. Dispositif de mesure, comprenant :
- un capteur (101) configuré pour mesurer une caractéristique d'un gaz, la caractéristique étant la concentration du gaz dans le sol d'une région,
- un module (103) de communication sans-fil configuré pour émettre la caractéristique mesurée du gaz,
- une horloge (112),
**caractérisé en ce que** le dispositif est configuré pour :
mesurer ladite caractéristique du gaz, ladite mesure étant déclenchée par l'horloge à un instant de mesure commun à une pluralité de dispositifs de mesure, transmettre, dans une plage horaire prédéterminée spécifique au dispositif de mesure, ladite caractéristique mesurée du gaz et son heure de transmission à destination d'un concentrateur (500).

2. Dispositif selon la revendication 1, le dispositif de mesure comprenant un module de contrôle (102) du capteur configuré pour que le capteur mesure la caractéristique mesurée du gaz pendant une plage de mesure prédéterminée.

3. Dispositif selon la revendication 1 ou la revendication 2, dans lequel le module de communication sans-fil est configuré pour émettre ladite caractéristique mesurée pendant une plage d'émission prédéterminée.

4. Dispositif selon l'une des revendications 1 à 3, dans lequel ladite horloge (112) est configurée pour être synchronisée sur la base d'un message émis par le concentrateur et reçu par le module de communication sans-fil.

5. Dispositif selon l'une quelconques des revendications 1 à 4, comportant : une canne tubulaire (205, 302, 401) équipée d'un ou de plusieurs orifices (206) traversant la paroi de la canne tubulaire, ledit un ou lesdits plusieurs orifices étant placés à proximité d'une extrémité (207) de la canne tubulaire destinée à être insérée dans le sol, et
- une pompe (105) pour aspirer le gaz présent dans le sol à travers le ou les orifices et ladite canne tubulaire, le gaz aspiré étant reçu par le capteur, et
dans lequel la canne a une longueur supérieure à 10 ou 70 centimètres, et inférieure 1, 3, ou 10 mètres, et
dans lequel la canne comprend entre 10 et 100 orifices traversant la paroi de la canne.

6. Dispositif selon la revendication 5, dans lequel le ou les orifices ont un diamètre compris entre 1 et 3 millimètres, sont espacés les uns par rapport aux autres d'au moins une distance comprise entre 1 et 3 millimètres, par exemple 2 millimètres, et sont agencés dans une portion de la canne s'étendant depuis ladite extrémité destinée à être insérée dans le sol sur toute la longueur de la canne ou sur une longueur comprise entre 3 millimètres et 20 centimètres.

7. Dispositif selon l'une quelconque des revendications 1 à 6, comprenant des filtres (110, 111) étanches aux liquides et perméables aux gaz agencés de part et d'autre dudit capteur.

8. Dispositif selon l'une quelconque des revendications 1 à 7, dans lequel le capteur est configuré pour détecter une concentration de dihydrogène.

9. Dispositif selon l'une quelconque des revendications 1 à 8, comprenant un module (104) d'alimentation en énergie électrique.

10. Ensemble comprenant au moins deux dispositifs selon l'une quelconque des revendications 1 à 9 et un concentrateur.

11. Installation comprenant, au sein d'une région, une pluralité de dispositifs selon l'une quelconque des revendications 1 à 9 en des emplacements respectifs de la région.

12. Installation selon la revendication 11, dans laquelle les dispositifs sont équipés d'horloges, et d'un module de contrôle de leur capteur configuré pour que les capteurs de tous les dispositifs mesurent la caractéristique mesurée du gaz pendant une même plage de mesure prédéterminée.

13. Installation selon l'une quelconque des revendications 11 à 12 dans laquelle les dispositifs sont équipés d'horloges, les modules de communication sans-fil respectifs étant configurés pour émettre ladite caractéristique mesurée à des plages d'émission prédéterminées.

14. Installation selon l'une quelconque des revendications 11 à 13, comprenant en outre un concentrateur.

## Patentansprüche

1. Messvorrichtung, umfassend:
- einen Sensor (101), der konfiguriert ist, um eine Eigenschaft eines Gases zu messen, wobei die Eigenschaft die Konzentration des Gases in dem Boden einer Region ist,
- ein drahtloses Kommunikationsmodul (103), das konfiguriert ist, um die gemessene Eigenschaft des Gases zu emittieren,
- einen Zeitgeber (112),
**dadurch gekennzeichnet, dass** die Vorrichtung zu Folgendem konfiguriert ist: Messen der Eigenschaft des Gases, wobei die Messung durch den Zeitgeber zu einem Messzeitpunkt ausgelöst wird, der einer Vielzahl von Messvorrichtungen gemeinsam ist,
Übertragen, innerhalb eines vorbestimmten Zeitfensters, das für die Messvorrichtung spezifisch ist, der gemessenen Eigenschaft des Gases und ihre Übertragungszeit an einen Konzentrator (500).

2. Vorrichtung nach Anspruch 1, die Messvorrichtung umfassend ein Steuermodul (102), des Sensors, das konfiguriert ist, damit der Sensor die gemessene Eigenschaft des Gases während eines vorbestimmten Messbereichs misst.

3. Vorrichtung nach Anspruch 1 oder 2, wobei das drahtlose Kommunikationsmodul konfiguriert ist, um die gemessene Eigenschaft während eines vorbestimmten Emissionsbereichs zu emittieren.

4. Vorrichtung nach einem der Ansprüche 1 bis 3, wobei der Zeitgeber (112) konfiguriert ist, um basierend auf einer Nachricht synchronisiert zu werden, die von dem Konzentrator emittiert und von dem drahtlosen Kommunikationsmodul empfangen wird.

5. Vorrichtung nach einem beliebigen der Ansprüche 1 bis 4, umfassend: ein rohrförmigen Stab (205, 302, 401), der mit einer oder mehreren Öffnungen (206) ausgestattet ist, die durch die Wand des rohrförmigen Stabs verlaufen, wobei die eine oder die mehreren Öffnungen in der Nähe eines Endes (207) des rohrförmigen Stabs angeordnet sind, der in den Boden eingeführt werden soll, und
- eine Pumpe (105) zum Ansaugen von Gas aus dem Boden durch die Öffnung(en) und den rohrförmigen Stab, wobei das angesaugte Gas von dem Sensor empfangen wird, und
wobei der Stab eine Länge von mehr als 10 oder 70 Zentimetern und weniger als 1, 3, oder 10 Metern aufweist, und
wobei der Stab zwischen 10 und 100 Öffnungen aufweist, die durch die Wand des Stabs verlaufen.

6. Vorrichtung nach Anspruch 5, wobei die Öffnung(en) einen Durchmesser zwischen 1 und 3 Millimetern aufweisen, um mindestens einen Abstand zwischen 1 und 3 Millimetern, z. B. 2 Millimetern, voneinander beabstandet sind und in einem Abschnitt des Stabs angeordnet sind, der sich von dem Ende, das in den Boden eingeführt werden soll, über die gesamte Länge des Stabs oder über eine Länge zwischen 3 Millimetern und 20 Zentimetern erstreckt.

7. Vorrichtung nach einem beliebigen der Ansprüche 1 bis 6, umfassend flüssigkeitsdichte und gasdurchlässige Filter (110, 111), die auf beiden Seiten des Sensors angeordnet sind.

8. Vorrichtung nach einem beliebigen der Ansprüche 1 bis 7, wobei der Sensor konfiguriert ist, um eine Konzentration von Dihydrogen zu erfassen.

9. Vorrichtung nach einem beliebigen der Ansprüche 1 bis 8, umfassend ein Modul (104) zur Versorgung mit elektrischer Energie.

10. Anordnung, umfassend mindestens zwei Vorrichtungen nach einem beliebigen der Ansprüche 1 bis 9 und einen Konzentrator.

11. Anlage, umfassend innerhalb einer Region eine Vielzahl von Vorrichtungen nach einem beliebigen der Ansprüche 1 bis 9 an jeweiligen Standorten in der Region.

12. Anlage nach Anspruch 11, wobei die Vorrichtungen mit Zeitgebern ausgestattet sind, und mit einem Steuermodul für ihren Sensor, das konfiguriert ist, damit die Sensoren aller Vorrichtungen die gemessene Eigenschaft des Gases während eines gleichen vorbestimmten Messbereichs messen.

13. Anlage nach einem beliebigen der Ansprüche 11 bis 12, wobei die Vorrichtungen mit Zeitgebern ausgestattet sind, wobei die jeweiligen drahtlosen Kommunikationsmodule konfiguriert sind, um die gemessene Eigenschaft zu vorbestimmten Emissionszeitpunkten zu emittieren.

14. Anlage nach einem beliebigen der Ansprüche 11 bis 13, ferner umfassend einen Konzentrator.

## Claims

1. A measurement device, comprising:
- a sensor (101) configured to measure a characteristic of a gas, the characteristic being the concentration of the gas in the soil of a region,
- a wireless communication module (103) configured to transmit the measured characteristic of the gas,
- a clock (112),
**characterized in that** the device is configured to:
measure said characteristic of the gas, said measurement being triggered by the clock at a measurement time common to a plurality of measurement devices,
transmit, within a predetermined time slot specific to the measurement device, said measured characteristic of the gas and its transmission time to a hub (500).

2. The device according to claim 1, the measurement device comprising a control module (102) of the sensor configured so that the sensor measures the measured characteristic of the gas during a predetermined measurement time slot.

3. The device according to claim 1 or claim 2, wherein the wireless communication module is configured to transmit said measured characteristic during a predetermined transmission time slot.

4. The device according to one of claims 1 to 3, wherein said clock (112) is configured to be synchronized based on a message emitted by the hub and received by the wireless communication module.

5. The device according to any one of claims 1 to 4, including: a tubular rod (205, 302, 401) equipped with one or more orifices (206) passing through the wall of the tubular rod, said one or more orifices being placed close to one end (207) of the tubular rod, intended to be inserted into the soil, and
- a pump (105) to draw the gas present in the soil through the orifice(s) and said tubular rod, the drawn gas being received by the sensor, and
wherein the rod has a length greater than 10 or 70 centimeters, and less than 1, 3, or 10 meters, and
whereinthe rod comprises between 10 and 100 orifices passing through the wall of the rod.

6. The device according to claim 5, wherein the orifice(s) have a diameter between 1 and 3 millimeters, are spaced each relative to the others by at least a distance between 1 and 3 millimeters, for example 2 millimeters, and are arranged in a portion of the rod extending from said end, intended to be inserted into the soil, over the entire length of the rod or over a length between 3 millimeters and 20 centimeters.

7. The device according to any one of claims 1 to 6, comprising filters (110, 111) liquid-tight and gas-permeable arranged on either side of said sensor.

8. The device according to any one of claims 1 to 7, in which the sensor is configured to detect a concentration of dihydrogen.

9. The device according to any one of claims 1 to 8, comprising a power supply module (104).

10. An assembly comprising at least two devices according to any one of claims 1 to 9 and a hub.

11. An installation comprising, within a region, a plurality of devices according to any one of claims 1 to 9 at respective locations of the region.

12. The installation according to claim 11,wherein the devices are equipped with clocks, and a control module of their sensor configured so that the sensors of all the devices measure the measured characteristic of the gas during the same predetermined measurement time slot.

13. The installation according to any one of claims 11 to 12, wherein the devices are equipped with clocks, the respective wireless communication modules being configured to transmit said measured characteristic at predetermined transmission time slots.

14. The installation according to any one of claims 11 to 13, further comprising a hub.
